# EUROPEAN PATENT APPLICATION

(11) **EP 1 233 021 A2**
(43) Date of publication of application: **21.08.2002**
(21) Application number: 02250923.6
(22) Date of filing: 11.02.2002
(51) Int. Cl.: C07K 5/093, A61K 38/06, A61P 25/28

(54) **An inhibitor of Beta amyloid cleavage enzyme**

(30) Priority: 20.02.2001 US 270006 P
(71) Applicant: Pfizer Products Inc., Groton, Connecticut 06340 (US)
(72) Inventor: Boyd, James Gorham, Pfizer Global Research & Dev., Groton, Connecticut 06340 (US); Singleton, David H., Pfizer Global Research & Dev., Groton, Connecticut 06340 (US)
(74) Representative: Motion, Keith Robert

(57) **Abstract**

This invention relates to a novel inhibitor of beta amyloid cleavage enzyme (BACE, transmembrane aspartyl protease beta-secretase, beta site APP cleavage enzyme, memapsin-2, BACE-1), pharmaceutical compositions containing it and its use in the treatment of neurological disorders such as Alzheimer's disease, Crutzfield-Jacob's disease, prion disorders, amyotrophic lateral sclerosis, progressive supranuclear palsy, head trauma, stroke, down's syndrome, pancreatitis, inclusion body myocitis, other peripheral amyloidoses and diabetes.

## Description

This invention relates to a novel inhibitor of beta amyloid cleavage enzyme (BACE, transmembrane aspartyl protease beta-secretase, beta site APP cleavage enzyme, memapsin-2, BACE-1), pharmaceutical compositions containing it and its use in the treatment of neurological disorders such as Alzheimer's disease, Crutzfield-Jacob's disease, prion disorders, amyotrophic lateral sclerosis, progressive supranuclear palsy, head trauma, stroke, Down's syndrome, pancreatitis, inclusion body myocitis, other peripheral amyloidoses and diabetes.

Alzheimer's disease (AD), a progressive neurodegenerative disease of the central nervous system (specifically brain) is characterized by gradual loss of memory, declining orientation skills, motor, sensory, linguistic and cognitive functions of the brain. The pathological indicators of the disease are neurofibrillary tangles and amyloid plaques. These amyloid plaques are unique to AD, however neurofibrillary tangles are associated with many dementia disorders. Beta amyloid peptide (βAP), a highly insoluble peptide 39-43 amino acids in length has a strong propensity to adopt beta sheet structures, oligomerize and form protein aggregates, is a primary component of amyloid plaques. Production of βAP occurs when amyloid polypeptide precursor is cleaved by certain proteases, a group known as secretases. Cleavage by β-secretase at the amino terminus of beta amyloid peptide and cleavage by γ-secretase between residues 39 and 43 (most often at residue 42) constitute the means by which this peptide is produced. Cleavage by α-secretase (and other metalloproteases) affords a soluble cleavage product by cleaving between residues 16 and 17 of the beta amyloid peptide. This pathway reduces the potential accumulation of βAP by producing a soluble product.

Neurofibrillary tangles are composed of microtubules and microfilaments once capable of transporting nutrients to nerve cells that have since degenerated into dysfunctional tangles. Primary components of these are highly phosphorylated tau proteins and βAP. These hyper-phosphorylated tau proteins are also associated with numerous neurodegenerative disorders including AD. βAP has been indicated as being responsible for the hyper phosphorylation of tau, thereby causing these neurofibrillary tangles.

Agents lowering concentration of βAP by limiting its production (inhibition of β or γ secretase) or increasing its clearance (stimulation of α-secretase) could have marked effects on AD and other neurodegenerative conditions. Specifically, pharmaceutical agents designed to inhibit β-secretase should reduce βAP concentrations, thereby reducing the propensity for amyloid plaques and neurofibrillary tangles.

### Summary of the Invention

This invention relates to a compound of the formula 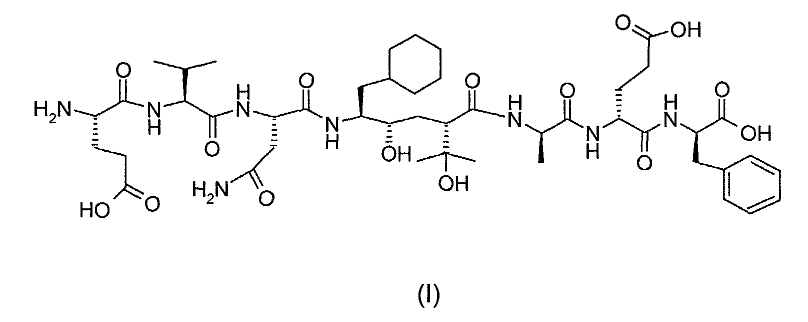 and pharmaceutically acceptable salts of such compound.

This invention also relates to a pharmaceutical composition for treating a disorder or condition selected from Alzheimer's disease, Crutzfield-Jacob's disease, prion disorders, amyotrophic lateral sclerosis, progressive supranuclear palsy, head trauma, stroke, Down's syndrome, pancreatitis, inclusion body myocitis, other peripheral amyloidoses and diabetes in a mammal, including a human, comprising an amount of a compound of the formula I, or a pharmaceutically acceptable salt thereof, that is effective in treating such disorder or condition, and a pharmaceutically acceptable carrier.

This invention relates to a method of treating a disorder or condition selected from Alzheimer's disease, Crutzfield-Jacob's disease, prion disorders, amyotrophic lateral sclerosis, progressive supranuclear palsy, head trauma, stroke, Down's syndrome, pancreatitis, inclusion body myocitis, other peripheral amyloidoses and diabetes in a mammal, including a human, comprising administering to said mammal an amount of a compound of the formula I, or a pharmaceutically acceptable salt thereof, that is effective in treating such disorder or condition.

The term "treating", as used herein, refers to reversing, alleviating, inhibiting the progress of, or preventing the disorder or condition to which such term applies, or one or more symptoms of such disorder or condition.

The term "treatment", as used herein, refers to the act of treating, as "treating" is defined immediately above.

Compounds of formula I may contain chiral centers and therefore may exist in different enantiomeric and diastereomeric forms. This invention relates to all optical isomers and all stereoisomers of compounds of the formula I, both as racemic mixtures and as individual enantiomers and diastereoisomers of such compounds, and mixtures thereof, and to all pharmaceutical compositions and methods of treatment defined above that contain or employ them, respectively.

As the compounds of formula I of this invention possess at least two asymmetric centers, they are capable of occurring in various stereoisomeric forms or configurations. Hence, the compounds can exist in separated (+)- and (-)-optically active forms, as well as mixtures thereof. The present invention includes all such forms within its scope. Individual isomers can be obtained by known methods, such as optical resolution, optically selective reaction, or chromatographic separation in the preparation of the final product or its intermediate.

In so far as the compounds of formula I of this invention are basic compounds, they are all capable of forming a wide variety of different salts with various inorganic and organic acids. Although such salts must be pharmaceutically acceptable for administration to animals, it is often desirable in practice to initially isolate the base compound from the reaction mixture as a pharmaceutically unacceptable salt and then simply convert to the free base compound by treatment with an alkaline reagent and thereafter convert the free base to a pharmaceutically acceptable acid addition salt. The acid addition salts of the base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained. The acids which are used to prepare the pharmaceutically acceptable acid addition salts of the aforementioned base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, p-toluenesulfonate and pamoate (i.e., 1,1'-methylene-bis-(2-hydroxy-3-naphthoate))salts.

In so far as the compounds of formula I of this invention are acidic compounds, they are also capable of forming a wide variety of different salts with various inorganic and organic acids. The chemical bases that are used as reagents to prepare the pharmaceutically acceptable base salts of this invention are those that form non-toxic base salts with the compound of formula I. Such non-toxic base salts include those derived from such pharmacologically acceptable cations as sodium, potassium, calcium and magnesium, etc.

For a review on pharmaceutically acceptable salts, see Berge et al., J. Pharm. Sci., 66, 1-19 (1977).

The present invention also includes isotopically labelled compounds, which are identical to those recited in formula I, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁵Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I of this invention and prodrugs thereof can generally be prepared by carrying out the procedures disclosed in the Schemes and/or in the Examples and Preparations below, by substituting a readily available isotopically labelled reagent for a non-isotopically labelled reagent.

A more specific embodiment of this invention relates to the above method wherein the disorder or condition being treated is Alzheimer's disease.

Another more specific embodiment of this invention relates to the above method wherein the disorder or condition being treated is amyotrophic lateral sclerosis.

Another more specific embodiment of this invention relates to the above method wherein the disorder or condition being treated is diabetes.

Another more specific embodiment of this invention relates to the above method wherein the disorder or condition being treated is pancreatitis.

Another more specific embodiment of this invention relates to the above method wherein the disorder or condition being treated is inclusion body myocitis.

Another more specific embodiment of this invention relates to the above method wherein the disorder or condition being treated is progressive supranuclear palsy.

Another more specific embodiment of this invention relates to the above method wherein the disorder or condition being treated is head trauma.

Another more specific embodiment of this invention relates to the above method wherein the disorder or condition being treated is stroke.

Another more specific embodiment of this invention relates to the above method wherein the disorder or condition being treated is a prion disorder.

Another more specific embodiment of this invention relates to the above method wherein the disorder or condition being treated is Crutzfield-Jacob's disease.

Another more specific embodiment of this invention relates to the above method wherein the disorder or condition being treated is Down's syndrome.

Another more specific embodiment of this invention relates to the above method wherein the disorder or condition being treated is selected from vascular dementia, dementia due to HIV disease, dementia due to head trauma, dementia due to Parkinson's disease, dementia due to Huntington's disease, dementia due to Pick's disease, dementia due to Crutzfield-Jacob's disease, substance-induced persisting dementia, dementia due to multiple etiologies and dementia not otherwise specified (NOS).

Another more specific embodiment of this invention relates to the above inventive method wherein the disorder or condition being treated is a dementia of the Alzheimer's type and is selected from the group consisting of dementia of the Alzheimer's type with early onset uncomplicated, dementia of the Alzheimer's type with early onset with delusions, dementia of the Alzheimer's type with early onset with depressed mood, dementia of the Alzheimer's type with late onset uncomplicated, dementia of the Alzheimer's type with late onset with delusions and dementia of the Alzheimer's type with late onset with depressed mood.

### Detailed Description of the Invention

The compound of formula I can be synthesized by using the solid phase peptide synthesis procedure illustrated in Scheme 1 below.

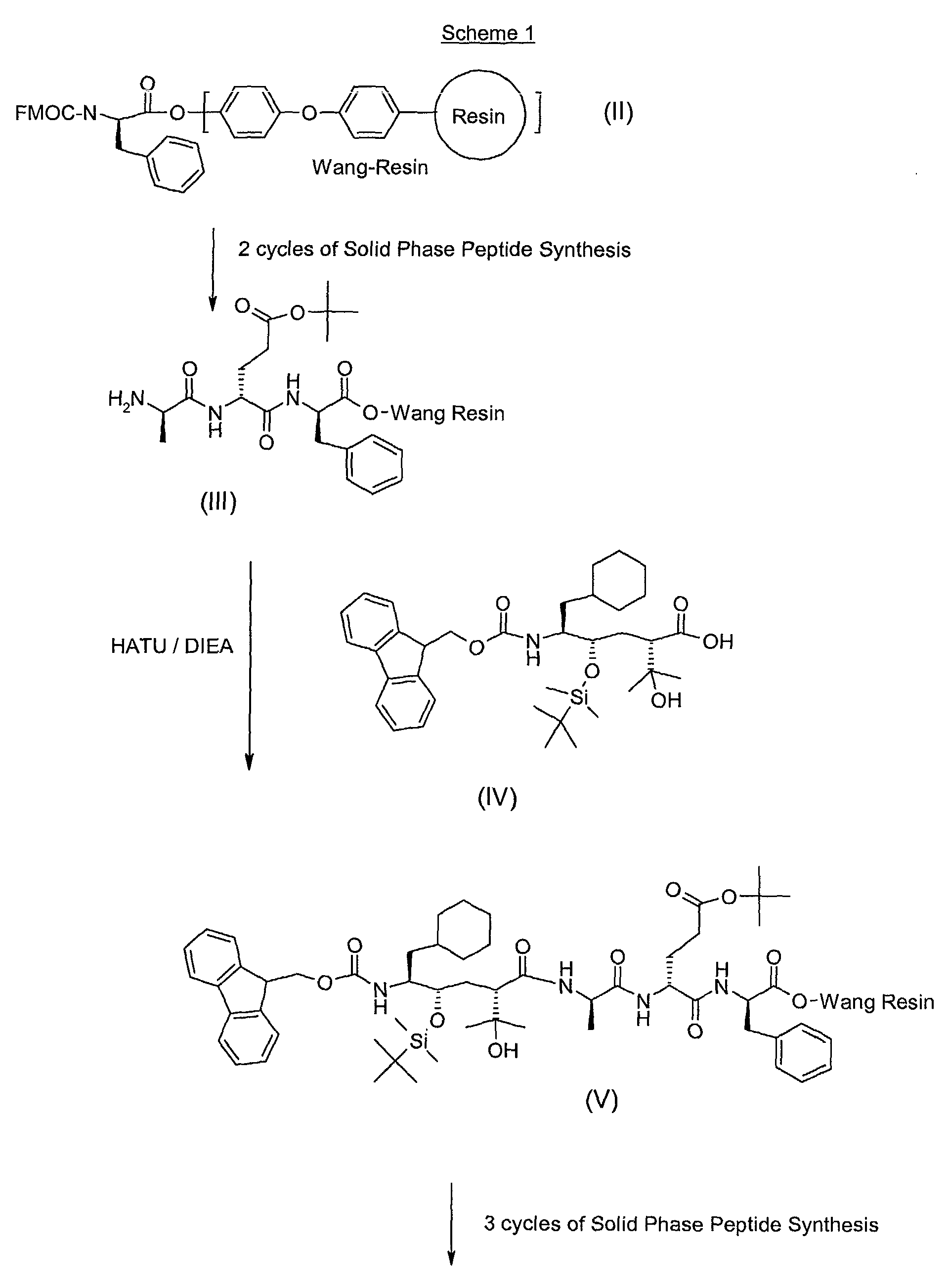

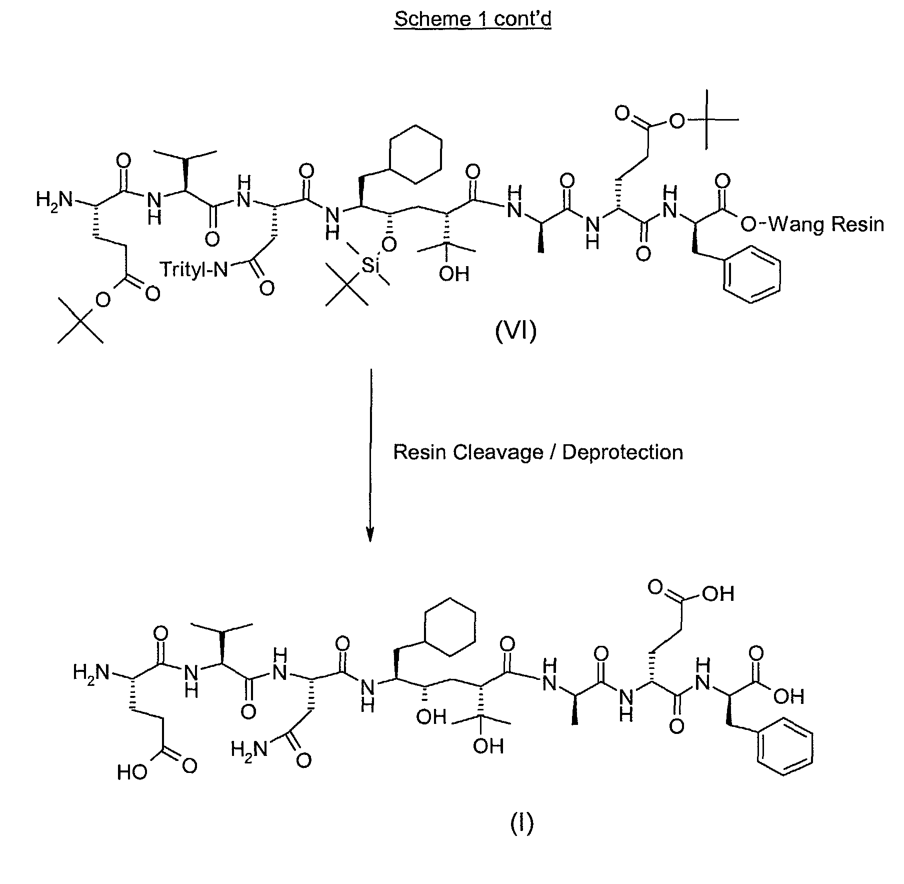

Referring to Scheme 1, FMOC-Phenylalanine Wang Resin (FMOC = 9-fluoroenylmethoxycarbonyl) is elaborated to the tri-peptide stage using Solid Phase Peptide Synthesis protocols optimized for efficiency of yield and time. These cycles, which are fully described in Examples 1 - 3, incorporate 2 FMOC deprotections, washes, a single coupling of 2-(1-H-Benzotriazole-1-yl)1,1,3,3-tetramethylurourium hexafluorophosphate (HBTU) activated amino acid, additional washes, capping and, finally, washing first with NMP then with 1:1 trifluoroethanol/dichloromethane. These washes help to relax resin secondary structure allowing for thorough deprotection and efficient coupling of the next incoming amino acid during the next cycle. Capping is suspended following incorporation of the isopropanol cyclohexyl homostatine derivative of formula IV, due to it's ability to acetylate an unprotected oxygen, rather than nitrogen, giving rise to chain ambiguity. The compound of formula IV can be synthesized using the procedure illustrated in Scheme 2 below. This procedure is also fully described in Examples 1 - 3.

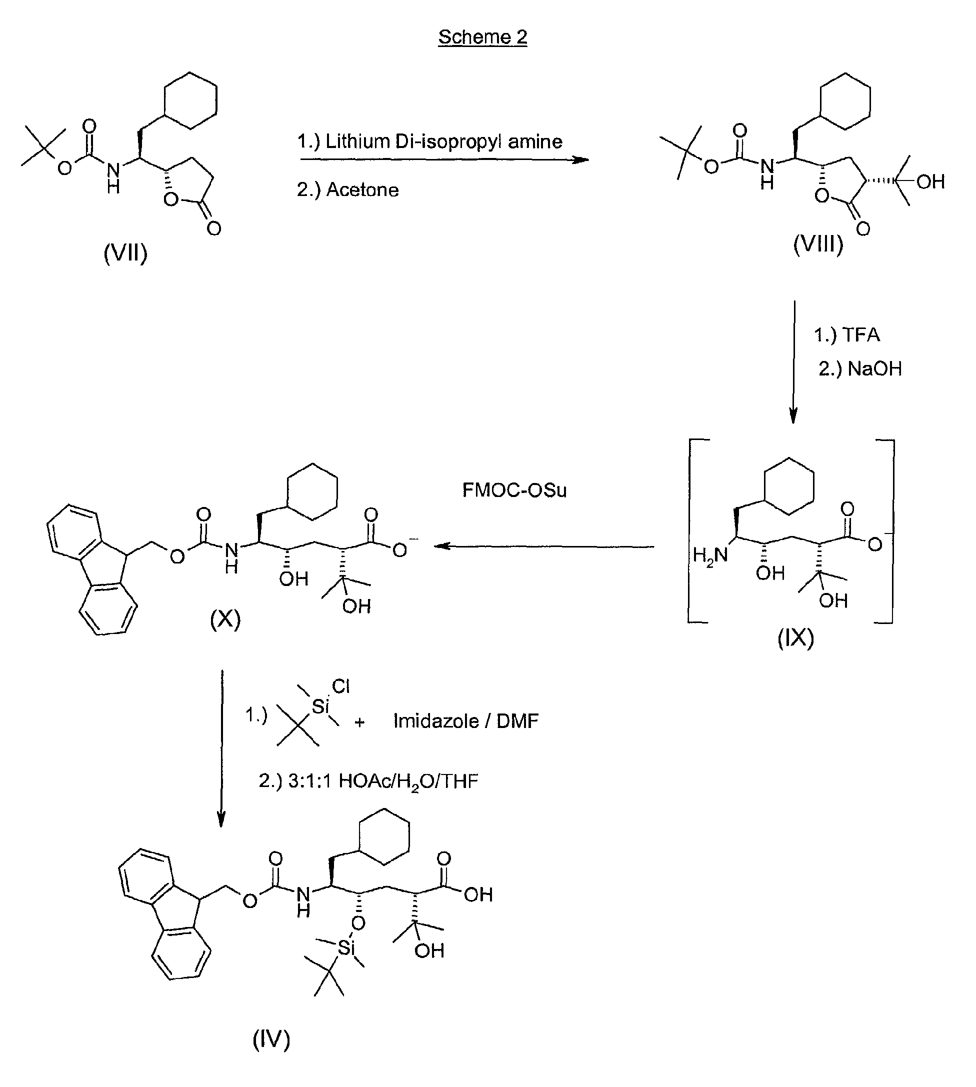

The cyclohexyl hyrdroxyethylene isopropanol dipeptide isostere IV is synthesized from the lactone of formula VII by aldol condensation of the lithium anion with acetone. Elaboration into the FMOC protected amino acid was performed in concurrent steps without isolating the intermediate stage products. Protection of the transition state alcohol with tertButyldimethylsilyl group, followed by hydrolysis of any esterification by products provides the dipeptide isostere IV suitable for peptide synthesis.

The pharmaceutically acceptable acid addition salts of the compound of the formula I are prepared in a conventional manner by treating a solution or suspension of the free base (I) with about one chemical equivalent of a pharmaceutically acceptable acid. Conventional concentration and recrystallization techniques are employed in isolating the salts. Illustrative of suitable acids are the acetic, hydrochloric, hydrobromic, hydroiodic, nitric, sulfonic, sulfuric, isonicotinic, lactic, salicylic, citric, tartaric, pantothenic, bitartaric, ascorbic, succinic, maleic, fumaric, glucaronic, saccharate, formate, benzoate, glutamate, methanesulfonic, ethanesulfonic, benzenesulfonic, p-toluenesulfonic, and gluconic acids.

The compound of formula I is a peptide based molecule designed to bind to β-Secretase. The peptide may be prepared as a pharmaceutically acceptable salt, or it may exist in its native charge state. It is based on the sequence of native APP; however, because it contains the hydroxyethylene bond isostere, is not capable of being hydrolyzed by the enzyme, thereby inhibiting cleavage of APP to βAP. It binds to the enzyme with IC₅₀ equal to 49x10⁻⁹M, as measured by in-vitro ELISA assays. Such assays, which are well known in the art, measure the concentration of βAP produced (by detection of an antibody against the amino terminus of βAP) in the presence of β-Secretase and APP versus control experiments in the absence of inhibitor.

The compound of formula I and its pharmaceutically acceptable salts (referred to collectively, hereinafter, as "the active compounds of this invention"), can be administered to a human subject either alone, or, preferably, in combination with pharmaceutically acceptable carriers or diluents, in a pharmaceutical practice. Such compounds can be administered orally or parenterally. Parenteral administration includes especially intravenous and intramuscular administration. Additionally, in a pharmaceutical composition comprising an active compound of this invention, the weight ratio of active ingredient to carrier will normally be in the range from 1:6 to 2:1, and preferably 1:4 to 1:1. However, in any given case, the ratio chosen will depend on such factors as the solubility of the active component, the dosage contemplated and the precise route of administration.

The active compounds of this invention can be administered to mammals via either the oral, transdermal, pulmonary, mucosal, or parenteral routes when dissolved in a pharmaceutically acceptable vehicle. The vehicle is dependent upon the route of administration and may contain adjuvants necessary for stability, bioavailability or absorption of the target molecule. Treatment dosage concentrations and frequency of administration will be determined according to the indicated outcome as determined by a prescribing physician.

In general, these compounds are most desirably administered in doses ranging from about 0.1 to about 100 mg/kg per day, in single or divided doses (i.e., from 1 to 4 doses per day), although variations will necessarily occur depending upon the species, weight and condition of the subject being treated and the particular route of administration chosen. However, a dosage level that is in the range of about 1 mg to about 10 mg per kg of body weight per day is most desirably employed. Nevertheless, variations may occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such higher dose levels are first divided into several small doses for administration throughout the day.

The active compounds of this invention can be administered alone or in combination with pharmaceutically acceptable carriers or diluents by any of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral use in treating the various disorders and conditions referred to above, the active compounds of this invention can be administered, for example, in the form of tablets or capsules, or as an aqueous solution or suspension. Tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active compounds of this invention may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound of this invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art.

For intramuscular, parenteral and intravenous use, sterile solutions of the active ingredient can be prepared, and the pH of the solutions should be suitably adjusted and buffered. For intravenous use, the total concentration of solutes should be controlled to render the preparation isotonic.

The following example illustrates the synthesis of the compound of formula I.

### EXAMPLE 1

### Synthesis of the Compound of Formula VIII

This compound was synthesized similarly to the procedure of Nishi, T *et al*., *Chemistry Letters,* p1993-1996 (1989) as below.

To a flame dried flask under a passive dry nitrogen atmosphere, 700µl (5mmol) diisopropylamine was diluted into 25ml THF and the solution cooled to an internal temperature of -35°C. Two milliliters (5mmol) n-butyl lithium in hexanes was added dropwise at a rate maintaining the internal temperature below -35°C. After holding this at -78°C for 10 minutes, a solution of 650 mg (2mmol) of carbamic acid, [2-cyclohexyl-1-(tetrahydro-5-oxo-2-furanyl)ethyl]-, 1,1-dimethylethyl ester, [S-(R*,R*)]- (9Cl) (the compound of formula VII) dissolved in 15ml THF was added dropwise via syringe, while maintaining the internal temperature below -55°C. The reaction was equilibrated at -78°C and 175µl (acetone volume only). Acetone was added at a rate such that internal temperature was maintained below - 70°C. After 1 hour, the reaction was worked up by quenching via addition of 200ml water and warming to room temperature. This was extracted with 2x150ml ethyl acetate (EtOAc). The combined EtOAc layers were washed with 2x200ml H₂O, 1x100ml saturated (sat.) sodium chloride (NaCI), dried over magnesium sulfate (MgSO₄), filtered and concentrated in vacuo yielding crude compound VIII. Purification via Flash Column Chromatography on 80gm silicon dioxide (SiO₂) eluting with EtOAc/hexanes in a step gradient fashion with 5 column volumes of 20% EtOAc/Hexanes, followed with 10 column volumes of 25% EtOAc/Hexanes, and finally eluting with 5 column volumes 33% EtOAc/hexanes afforded separation of 2 closely eluting materials. Fractions corresponding to the less polar, more predominant isomer (as judged by thin layer chromatography (TLC)) were pooled, affording 467mg (63%) of the compound of formula VIII, H¹ NMR (CDCl₃) δ 4.47 (m, 1 H), 4.31 (d, j=9hz, 1H), 3.87 (m, 1H), 3.30 (br s, 1H), 2.72 (dd, j=10hz, 8hz, 1H), 2.37 (1H, dt, j=11Hz, 7hz, 1H), 2.11 (m, 1H), 1.78 (d, j=13hz, 1H), 1.66, (m, 6H), 1.42 (s, 10H), 1.23 (d, 9H), 0.95 (m, 1H), 0.83 (m, 1H); m/z calcd. 370.46, obs. 370.08

### EXAMPLE 2

### Synthesis of the Compound of Formula IV

76 mg (0.205mmol) of the title compound of Example 1 was dissolved in 3ml trifluoroacetic acid (TFA). After 5 minutes, the trifluoroacetic acid was removed in vaccuo. The resultant solid was dissolved in 2ml acetonitrile (CH₃CN), 800µl water (H₂O) and 200µl (1mmol) 5N sodium hydroxide (NaOH). This was observed to be the desired amino acid intermediate as judged by High Pressure Liquid Chromatography coupled with electrospray mass spectrometry detection (LC-ESMS) (m/z calcd.=288.4, obs. 288.0) after 30 minutes of reaction time. The pH was adjusted to 11 via addition of 1N hydrochloric acid (HCI) and buffered via addition of 200mg sodium bicarbonate (NaHCO₃). Seventy five milligrams (0.222mmol) 9-fluorenylmethyloxycarbonyl-N-hydroxysuccinimide (FMOC-OSu) was added. After 90 minutes, the product was isolated by diluting with 75ml EtOAc and 75ml H₂O. The EtOAc layer was re-extracted with 50ml 1N NaOH. The combined aqueous layers were washed with 1 x 50 ml additional EtOAc. The combined aqueous layer was adjusted to pH 1 via addition of 1N HCl and extracted with 2 x 50 ml EtOAc. These combined organic layers were washed with 1 x50 ml H₂O, 1 x 50 ml sat. sodium chloride (NaCI), dried over magnesium sulfate (MgSO₄), filtered and concentrated in vacuo yielding an oil. This crude material was used without further purification.

To this oily solid was added 1ml DMF followed by 450 mg (3mmol) tertbutyldimethylsilyl chloride and 100mg (1.5mmol) imidazole. The reaction was stirred overnight at ambient temperature. The product was isolated by addition of 10% citric acid and extracted with 2 x 50ml EtOAc. The combined EtOAc layers were washed with 1 x 50 ml H₂O, 1 x 50 ml saturated (sat.) NaCl, dried over MgSO₄, filtered and concentrated in vacuo to an oil. LC-ESMS indicated this material to be a mono silyl ether/silyl ester intermediate (M/z calc'd = 738.18, observed = 738.02). The ester was hydrolyzed by dissolving the ester in 2 ml 3:1:1 acetic acid (HOAc)/H₂O/tetrahydrfuran (THF). After stirring for 90 minutes, the reaction was concentrated in vacuo. Purification via Flash Column Chromatography on 25gm SiO₂ eluting with 33% EtOAc/hexanes and combining fractions corresponding to the most prevalent material (as judged by TLC) afforded 41mg (32%) of the compound of formula IV. ¹H NMR (CDCI3) δ 7.73(q, j=7hz, 2H), 7.54(d, j=11hz, 2H), 7.37(q, j=7Hz, 2H), 7.27(q, j=7hz, 2H), 4.76(d, 8.7Hz, 1H), 4.30(m, 4H), 3.74(m, 2H), 2.47(m, 1H), 1.84(m,1H), 1.67(m, 2H), 1.25(d,j=1Hz,10H), 1.23 (m,2H), 0.90 (s, 6H), 0.86 (s, 6H), 0.11 (d, j=3hz, 9H), m/z calcd. = 623.91, obs. = 624.15.

### EXAMPLE 3

### Synthesis of the Compound of Formula I

Peptide assembly was completed by solid phase peptide synthesis using modifications to manufacturer supplied (Applied Biosystems) FMOC (9-fluoroenylmethoxycarbonyl) based synthesis cycles. Our modified cycles deprotect the amino terminus with 2 x 5 minute treatments with 20% piperidine/NMP; the efficiency of which is monitored by UV absorbance at 301 nm by passage of a small aliquot of deprotection solution through a UV absorbance detector. In a separate cartridge, the incoming amino acid is activated with 0.9 equivalents each of 2-(1H-benzotriazole-1-yl)-1,1,3,3 tetramethyluronium hexafluorophosphate (HBTU)/1-hydroxybenzotriazole (HOBt) dissolved in dimethylformamide (DMF). Two equivalents of diisopropylethyl amine (DIEA) are added. Concurrently, the resin is washed with N-methylpyrrolidone (NMP) to remove deprotection by-products. The wash solution is drained from the resin and the activated amino acid ester is transferred to the resin and stirred to allow coupling to the amino terminus for 20 minutes. The residual coupling solution is drained and the resin washed again with NMP. To ensure peptide homogeneity, a solution of 0.4M acetic anhydride/0.04M (HOBt) in NMP and 12mmole DIEA are added to the resin to acetylate any potential unreacted sites. Finally, the resin is washed with NMP, drained, then washed with a mixture of 1:1 dichloromethane/2,2,2-trifluoroethanol and drained. This typifies one cycle of peptide synthesis.

Four hundred seventeen milligrams of FMOC-Phenylalanine-Wang-Resin (0.25mmol) was placed in a reaction vessel suitable for synthesis on an Applied Biosystems Peptide Synthesizer, model 433. The resin was washed twice each with dichloromethane, then with NMP. One millimole FMOC-Glu(OtBu) was coupled to the deprotected phenylalanine, followed by 1mmol FMOC-Ala using the above cycles. At the tripeptide stage, the resin was again deprotected using the same protocol above and synthesis terminated following the NMP wash without introduction of an additional amino acid. Seventy-five percent of the resin was removed and stored for future use. In a dry, stoppered erlenmeyer flask, forty-one milligrams (65.7µmol) of the title compound of Example 2 was dissolved in 500 µl DMF. To this was added 22.5mg (59.1 umol) N-[(dimethyamino)-1H-1, 2, 3-triazolo[4, 5-b]pyridino-1-ylmethylene]-N-methyl-methanaminium hexafluorophosphate N-oxide (HATU) followed by 59 µl 2M DIEA/NMP. To this, 25% of tripeptide resin (62.5µmol) was transferred and the reaction vortexed at ambient temperature for 60 minutes. The resin was filtered and washed with NMP, and then returned to the peptide synthesizer and peptide assembly continued. These additional cycles were performed in the absence of acetic anhydride capping due to potential instability. The completed peptide resin was washed with NMP, followed by dichloromethane (DCM) and dried in vacuo. Concurrent cleavage and deprotection of side chain groups was performed by treatment with 2ml Reagent K (King, D, *Int. J. Peptide Protein Res.,* 36, 255-266 (1990)). The product was isolated via filtration from the resin; and the filtrate added to 50 ml ethyl ether (Et₂O). The resulting white precipitate was collected by centrifugation, washed with 50 ml additional Et₂O, centrifuged, decanted and dried in vacuo, affording 25mg (41%) of crude compound I. Purification in 2 equal portions via semi-preparative reverse phase HPLC (Column = Phenomenex LUNA 5µm c5, 250 x 10mm, Flow = 3 ml/min, Gradient = 0% B to 80% B over 30 minutes (A=5% CH₃CN /0.1% TFA/94.9% H₂O; B=100% CH₃CN)), collecting fractions corresponding to the less polar peak, afforded 8.7mg (14%) of the purified compound of formula I. Re-analysis by reverse phase LC-ESMS reveals homogenous product (m/z calc'd = 977.13, observed = 977.02).

## Claims

1. A compound of the formula 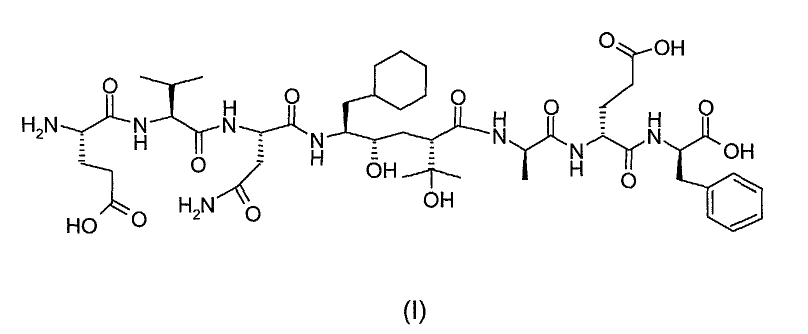 or a pharmaceutically acceptable salt thereof.

2. A pharmaceutical composition including a compound according to claim 1 or a pharmaceutically acceptable salt thereof, together with a pharmaceutically acceptable carrier or diluent.

3. A compound according to claim 1 or a pharmaceutically acceptable salt or composition thereof, for use as a medicament.

4. A pharmaceutical composition for treating a disorder or condition selected from Alzheimer's disease, Crutzfield-Jacob's disease, prion disorders, amyotrophic lateral sclerosis, progressive supranuclear palsy, head trauma, stroke, Down's syndrome, pancreatitis, inclusion body myocitis, other peripheral amyloidoses and diabetes in a mammal, including a human, comprising an amount of a compound according to claim 1, or a pharmaceutically acceptable salt thereof, that is effective in treating such disorder or condition, and a pharmaceutically acceptable carrier or diluent.

5. The use of a compound according to claim 1, or a pharmaceutically acceptable salt thereof, for the manufacture of a medicament for the treatment of a disorder or condition selected from Alzheimer's disease, Crutzfield-Jacob's disease, prion disorders, amyotrophic lateral sclerosis, progressive supranuclear palsy, head trauma, stroke, Down's syndrome, pancreatitis, inclusion body myocitis, other peripheral amyloidoses and diabetes in a mammal, including a human.

6. A use according to claim 5, wherein the disorder or condition being treated is Alzheimer's disease.

7. A use according to claim 5, wherein the disorder or condition being treated is Down's syndrome.

8. A use according to claim 5, wherein the disorder or condition being treated is amyotrophic lateral sclerosis.

9. A use according to claim 5, wherein the disorder or condition being treated is stroke.

10. A use according to claim 5, wherein the disorder or condition being treated is head trauma.

11. A use according to claim 5, wherein the disorder or condition being treated is a diabetes.

12. A use according to claim 5, wherein the disorder or condition being treated is pancreatis.

13. A use according to claim 5, wherein the disorder or condition being treated is inclusion body myocitis.

14. A use according to claim 5, wherein the disorder or condition being treated is a prion disorder.

15. A use according to claim 5, wherein the disorder or condition being treated is Crutzfield-Jacob's disease.

16. A use according to claim 5, wherein the disorder or condition being treated is progressive supranuclear palsy.

17. The use of a compound according to claim 1, or a pharmaceutically acceptable salt thereof, for the manufacture of a medicament for the treatment of vascular dementia, dementia due to HIV disease, dementia due to head trauma, dementia due to Parkinson's disease, dementia due to Huntington's disease, dementia due to Pick's disease, dementia due to Crutzfield-Jacob's disease, substance-induced persisting dementia, dementia due to multiple etiologies, and dementia not otherwise specified (NOS), or the disorder or condition is a dementia of the Alzheimer's type and is selected from the group consisting of dementia of the Alzheimer's type with early onset uncomplicated, dementia of the Alzheimer's type with early onset with delusions, dementia of the Alzheimer's type with early onset with depressed mood, dementia of the Alzheimer's type with late onset uncomplicated, dementia of the Alzheimer's type with late onset with delusions and dementia of the Alzheimer's type with late onset with depressed mood.
